# EUROPEAN PATENT APPLICATION

(11) **EP 2 270 003 A1**
(43) Date of publication of application: **05.01.2011**
(21) Application number: 10184131.0
(22) Date of filing: 10.02.2006
(51) Int. Cl.: C07D 405/06

(54) **Novel processes for the preparation of a 2H-chromene**

(30) Priority: 18.02.2005 WO PCT/EP2005/001695
(62) Divisional of application: 06706815.5
(71) Applicant: Acino Pharma AG, 4253 Liesberg (CH)
(72) Inventor: Schneider, Peter, 4103, Bottmingen (CH); Tahtaoui, Chouaib, 68170, Rixheim (FR)
(74) Representative: Vossius & Partner

(57) **Abstract**

The present invention relates to novel processes for the preparation of the compound of formula I (Iclaprim), related to dihydrofolate reductase inhibitors 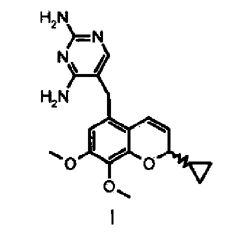 and to valuable intermediates of these processes.

## Description

### Field of the invention

The present invention relates to novel processes for the preparation of a compound of formula I (Iclaprim), related to dihydrofolate reductase inhibitors 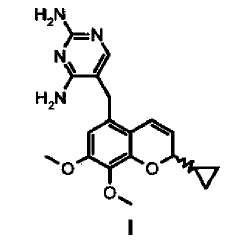 and to valuable intermediates of these processes.

### Background of the invention

The compound of formula I has valuable antibiotic properties. The compound can be used in the control or prevention of infectious diseases in mammals, both humans and non-humans. In particular, they exhibit pronounced antibacterial activity, even against multiresistant Gram-positive strains and against opportunistic pathogens such as e.g. *Pneumocystis carinii.* The compound can also be administered in combination with known substances of antibacterial activity and exhibit synergistic effects with some of them.

Typical combination partners are e.g. sulfonamides or other inhibitors of enzymes, which are involved in folic acid biosynthesis such as, for example, pteridine derivatives.

The synthesis of compound I as described in the US Patent Specification No. 5,773,446 and patent application PCT/EP 2004/008682 starts from relative expensive starting materials and the last steps of the synthesis are difficult to control.

Therefore, there is a need for a process for preparing compound I with a higher overall yield and reduced number of intermediates, which have to be isolated. The aim is a process where all isolated intermediates are crystalline and do not require chromatography. In addition this process allows to synthesize compounds related to the structure I from a common intermediate and a cheap starting material.

### Summary of the invention

The present invention provides a process for preparing the compound of the formula I from the intermediate of formula **6** and **12.**

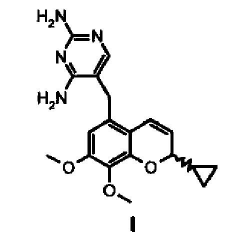

The intermediate of formula **3** is synthesized in 3 steps from a readily available starting material 1 (Scheme 1). The diamino pyrimidine substituent of **1** is selectively protected according to R.J. Griffin et al., J.Chem.Soc. Perkin Trans I, 1811 (1992) leading to compound of formula **2,** which in turn is formylated to a compound of formula **3** (Scheme 1).

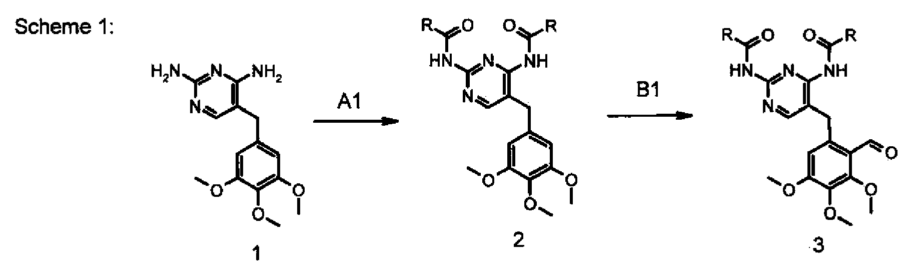

Another route of obtaining the compound of formula **3** is depicted in Scheme 2. Compound of formula **4** is protected at the diamino pyrimidine group to a compound of formula **5** followed by carbonylation of **5** to the intermediate of structure **3** (Scheme 2).

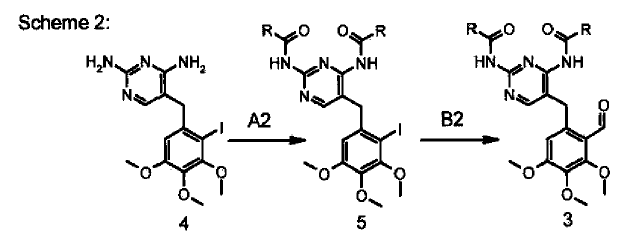

The compound of formula **3** is transformed by selective demethylation to the key intermediate of formula **6** (Scheme 3).

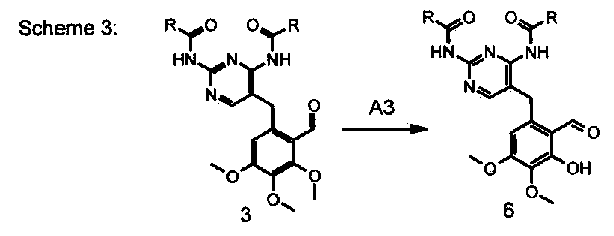

Condensation of compound of structure **6** after phenol protection **(7)** with ketone **8** resulted in formation of compound of formula **9.** Reduction to compound of structure **10** and acid or palladium catalyzed cyclisation, depending on the protecting group lead to compound of formula **11** as depicted in Scheme 4.

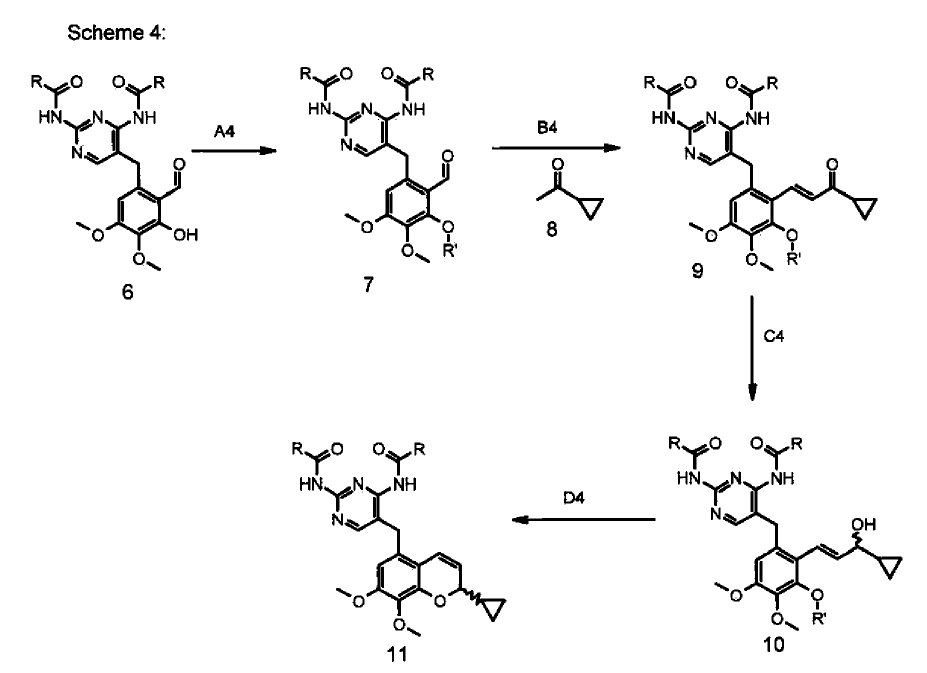

Alternatively, compound of structure **2** is acylated to compound of structure **12,** which is selectively demethylated to compound of structure **13** and reacted with a compound of formula **14** to obtain a compound of formula **15** (Scheme 5).

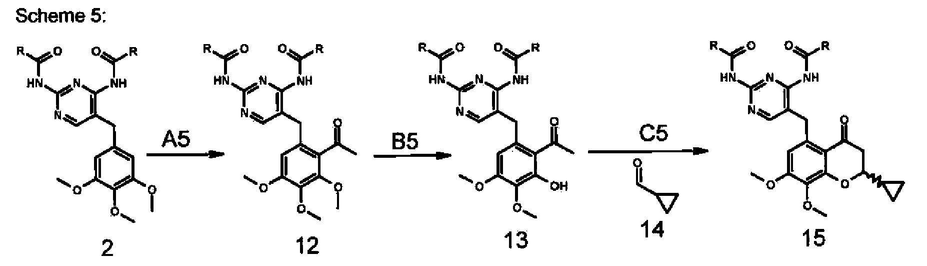

Compound of formula **17** can be synthesized either by acylation of compound of formula **2** with compound of formula **16** or starting from compound of formula **12** by reacting with the compound of formula **14.** Selective demethylation of compound of formula **17** by treatment with sodium iodide render the compound of formula **18** and subsequent cyclisation leads to a compound of formula **15** (Scheme 6).

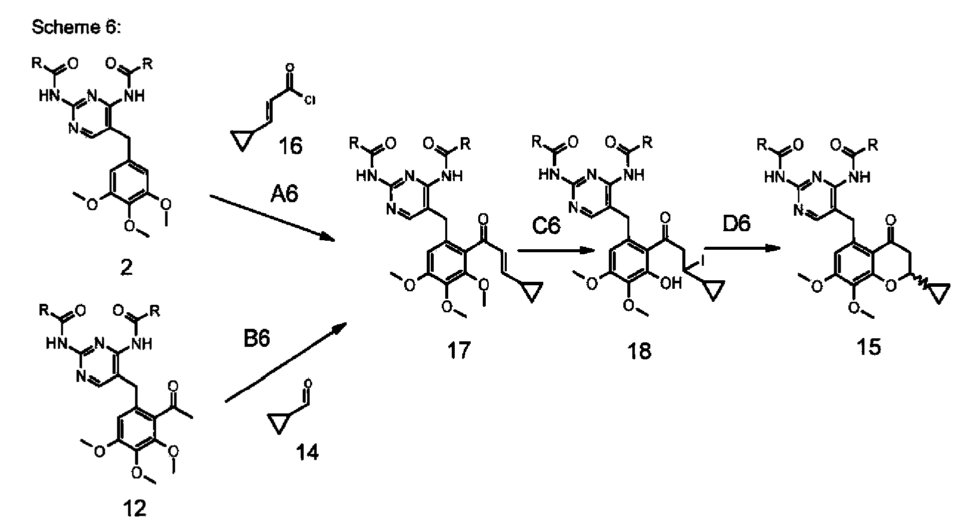

Compound of formula **15** is reduced to a compound of formula **19** and transformed into compound of formula **11** as shown in Scheme 7.

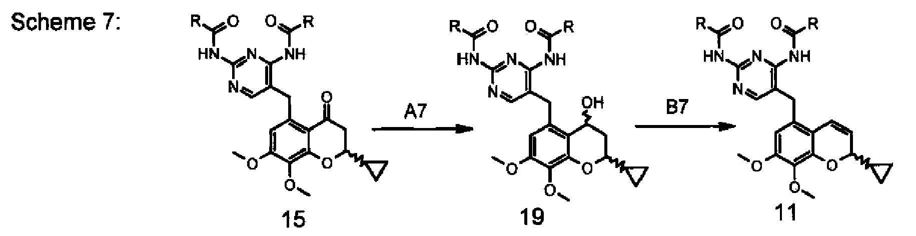

Deprotection of compound of formula **11** leads to the target compound of formula I as depicted in Scheme 8.

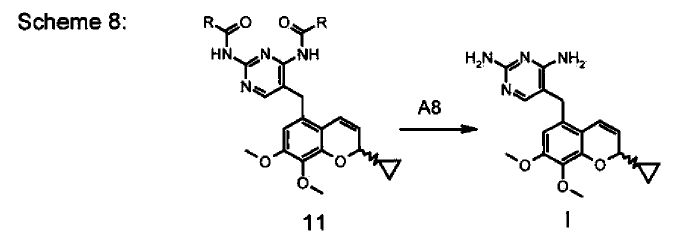

The compound of formula I is basic in nature and can be, if desired, transformed with an acid into pharmaceutically acceptable salts. Suitable acids are, e.g. hydrochloric acid, maleic acid, succinic acid, L(+)-lactic acid, DL-lactic acid, glycolic acid, 1-hydroxy-naphthalene-2-carboxylic acid, tartaric acid, citric acid, methane sulfonic acid. Most preferred are carboxylic acids.

### Detailed description of the invention

The process of the present invention provides many advantages and improvements over the current process of synthesizing compound of formula I as described in the US Patent Specification No. 5,773,446 and the patent application PCT/EP 2004/008682. The corresponding starting materials of formulae 1, **8** and **14** are commercially available in bulk quantities, whereas compound of formula **16** can be prepared from compound of formula **14** and malonic acid according the literature reference e.g. Z. Ma et al., Tetrahedron: Asymmetry 6 (6), 883 (1997). The central intermediate of formula **6** to prepare compound of formulae I may be prepared following the reaction sequences depicted in Schemes 1 to 3. The protection A1 of trimethoprim 1 can be done by heating compound of formula **1** with acid anhydrides, e.g. acetic anhydride, isobutyric acid anhydride or pivaloyl acid anhydride in an inert, high boiling solvent like toluene, p-xylene or in plain acid anhydride up to about 120 °C to 160 °C. The formulation B1 of the protected trimethoprim **2** can be achieved in an inert solvent, e.g. dichloromethane, dichloroethane, preferably dichloromethane with dichloromethyl-methyl ether and a Lewis acid, e.g. tin tetrachloride at 0 °C to -30 °C, preferably at -10 °C. Alternatively, compound of formula **3** can also be synthesized via protection A2 of compound **4** with acid anhydrides, e.g. acetic anhydride, methyl-propionic acid anhydride or pivaloyl acid anhydride in an inert, high boiling solvent like toluene, p-xylene or in plain acid anhydride, preferably methyl-propionic acid anhydride up to about 120 °C to 160 °C. Carbonylation B2 of compound of formula **5** can be effected in an inert atmosphere and solvent, e.g. tetrahydrofuran, with palladium tetrakis as catalyst, carbon monoxide and tri-butyl tin-hydride at 60 °C to 80 °C. The selective demethylation A3 can be done in an inert solvent, e.g. dichloromethane, acetonitrile, in combination with a Lewis acid like aluminium trichloride, boron trichloride, boron tribromide, manganese dichloride, manganese diiodide, preferably aluminium trichloride and a nucleophile, e.g. sodium iodide, dimethyl sulfide, diethyl sulfide, tetrahydrothiophene, preferably sodium iodide at room temperature up to 40 °C.

The synthesis of the target compound I starting from trimethoprim is shown in Schemes 1 to 8. Starting from intermediate with structure **6** by protection A4 of the phenol with e.g. chloromethyl-methylether, di-tertiary butyl methylchlorosilane, allyl halogenides, preferably with chloromethyl-methylether or 3-bromo-1-propene in an inert solvent like dichloromethane, tetrahydrofuran, dimethoxyethane, dimethyl-formamide, preferably tetrahydrofuran with a base like triethylamine, sodium hydride, potassium tertiary butoxide, preferably triethylamine or potassium tertiary butoxide at 0 °C to 40 °C preferably at 0 °C. The condensation B4 of 7 with acetyl cyclopropane in e.g. dichloromethane, tetrahydrofuran, dimethoxyethane, dimethyl-formamide, preferably tetrahydrofuran and a base like sodium hydride, tertiary amines, potassium tertiary butoxide preferably potassium tertiary butoxide at 0 °C to 40 °C preferably at ambient temperature leads to compound of formula **9.** Reduction C4 of compound of formula **9** to compound of formula **10** was achieved in e.g. isopropanol, tetrahydrofuran, dimethoxyethane, preferably in isopropanol or tetrahydrofuran with an alkali or metal borohydride at -10 °C up to ambient temperature, preferably at -10 °C up to 0 °C with sodium borohydride. The deprotection of the phenol followed by cyclisation D4 of compound of formula **10** was achieved in dichloromethane, tetrahydrofuran or acetonitrile with a palladium complex or an acid e.g. trifluoroacetic acid, para toluene sulfonic acid, methane sulfonic acid, hydrochloric acid, ammonium formiate, preferably with a palladium complex and ammonium formiate or trifluoroacetic acid depending on the nature of the protecting group at room temperature or up to 60 °C and leads to compound of formula **11.**

Friedel-Crafts acylation A5 of compound of formula **2** with an acylation agent like acetyl chloride or acetic acid anhydride, preferably acetic acid anhydride and a Lewis acid, e.g. aluminium trichloride, titanium tetrachloride or tin tetrachloride, preferably tin tetrachloride in an inert solvent, e.g. dichloromethane, dichloroethane at -10 °C to 40 °C, preferably at 0 °C up to room temperature leads to compound **12,** which in turn is demethylated B5 in an inert solvent, e.g. dichloromethane, acetonitrile, in combination with a Lewis acid like aluminium trichloride, boron trichloride, boron tribromide, preferably aluminium trichloride and a nucleophile, e.g. sodium iodide, dimethyl sulfide, tetrahydrothiophene, preferably sodium iodide at room temperature up to 40 °C leading to compound of formula **13.** Condensation C5 of compound of formula **13** with compound of formula **14** in acetonitrile and pyrrolidine as base at room temperature up to 50 °C, preferably room temperature leads to compound of formula **15.**

Acylation A6 of compound of formula **2** with compound of formula **16** under Friedel-Crafts conditions with a Lewis acid, e.g. aluminium trichloride, titanium tetrachloride or tin tetrachloride, preferably tin tetrachloride in an inert solvent, e.g. dichloromethane, dichloroethane at -10 °C to 40 °C, preferably at 0 °C up to room temperature leads to compound of formula **17,** which also can be synthesised by the condensation B6 of **12** with compound of formula **14** in tetrahydrofuran and potassium tertiary butoxide as base at room temperature up to 50 °C, preferably room temperature. Demethylation C6 of **17** in an inert solvent, e.g. dichloromethane, acetonitrile, in combination with a Lewis acid like aluminium trichloride, boron trichloride, boron tribromide, preferably aluminium trichloride and a nucleophile, e.g. sodium iodide, dimethyl sulfide, tetrahydrothiophene, preferably sodium iodide at room temperature up to 40 °C leading to compound of formula **18.** Cyclisation D6 of compound of formula **18** can be achieved in an inert solvent like dimethylformamide and potassium carbonate as base at room temperature leading to compound of formula **15.**

The reduction A7 of compound of formula **15** to compound of formula **19** was achieved in e.g. isopropanol, tetrahydrofuran, dimethoxyethane, preferably in isopropanol with sodium borohydride at 0 °C up to ambient temperature, preferably at ambient temperature. Water elimination B7 of compound of formula **19** with an acid like hydrochloric acid, methane sulfonic acid, trifluoro-acetic acid, preferably trifluoroacetic acid in toluene at room temperature up to 100 °C, preferably at 80 °C leads to compound of formula **11.**

Deprotection A8 of compound of formula **11** can be achieved in a mixture of organic solvents, e.g. tetrahydrofuran, methyl alcohol, preferably tetrahydrofuran and water with a strong base like sodium or potassium hydroxide, preferably sodium hydroxide at 40 °C to 80 °C preferably at 50 °C leading to the target compound 1.

The compounds of formulae **2, 3, 5, 6, 7, 9** to **13, 15,** and **17** to **19** are novel and are also objects of the invention. They can be prepared according to the reaction sequences elucidated in Schemes 1 to 8. The preparation of compounds outlined in Schemes 1 to 8 are, moreover, described in more detail in the examples.

As already mentioned, the compound of formula I or their pharmaceutical acceptable salts have valuable antibacterial properties. These compounds are active against a large number of pathogenic microorganisms such as e.g. *S. aureus, P. carinii* etc. by virtue of their activity in inhibiting bacterial dihydrofolate reductase (DHFR). The activity of compound I is described in more detail in P.G. Hartmann et al. Abstracts, F2020, 42nd Interscience Conference on Antimicrobial Agents and Chemotherapy, San Diego, Ca, Sep 27-30, 2002; American Society for Microbiology: Washington, DC, 2002.

Additional objects, advantages, and novel features of this invention will become apparent to those skilled in the art upon examination of the following examples, which are not intended to be limiting the scope of the invention.

The following examples illustrate the invention in more detail. Examples 1 to 11 describe the preparation of compound **6,** while examples 12 to 19 describe the preparation of the compound of formula **11** from compound of formula **6.** Examples 20 to 26 describe the synthesis of compound **15** which is transformed to compound **11** as described in the examples 27 and 28, and examples 29 and 30 describe the transformation of compound **11** to the end product of formula I (Iclaprim).

### Examples

Compound of formula **4** can be prepared e.g. according to M. Calas et al., Eur.J.Med.Chem.Chim.Ther., 17 (6), 497 (1982). Compound **7** can be prepared in analogy to e.g. W.B. Wright et al., J.Med.Chem., 11(6), 1164 (1968). The compound of formula **27** can be synthesized e.g. according to Z. Ma et al., Tetrahedron: Asymmetry 6 (6), 883 (1997).

All other reagents and solvents are readily commercially available, for example from Fluka or equivalent commercial suppliers. The temperatures are given in degrees Celsius.

### LCMS System

**HPLC Column 01:** Reverse Phase, *Atlantis* dC₁₈ 3 µm 4.6x75 mm column with guard catridge

**Gradient 01:**

| Time Min. | Flow mL | %A water / 10 mM Formic acid | %B Acetonitrile |
|---|---|---|---|
| 0.00 | 0.7 | 95 | 5 |
| 1.00 | 0.7 | 87 | 13 |
| 2.00 | 0.7 | 79 | 21 |
| 5.00 | 0.7 | 55 | 45 |
| 7.00 | 0.7 | 32 | 68 |
| 10.00 | 0.7 | 5 | 95 |
| 12.00 | 0.7 | 5 | 95 |
| 13.00 | 0.7 | 95 | 5 |

**HPLC Column 02:** Reverse Phase, *Waters* C₁₈ 3.5 µm 3x20 mm column **Gradient 02:**

| Time Min. | Flow mL | %A water / 10 mM Formic acid | %B Acetonitrile |
|---|---|---|---|
| 0.00 | 0.7 | 95 | 5 |
| 3.00 | 0.7 | 5 | 95 |
| 3.50 | 0.7 | 5 | 95 |
| 3.60 | 0.7 | 95 | 5 |
| 4.50 | 0.7 | 95 | 5 |

**Solvent A:** 10 mM Formic acid (Formic acid 377 µl) was added to HPLC grade water (1 L, *Millipore* filtered)

**Solvent B:** Acetonitrile HPLC grade (*Biosolve Ltd*)

**Wavelength:** 210 nm to 400 nm.

**HPLC Apparatus Type:** *Finnigan Surveyor* LC pump, *Finnigan Surveyor* Photodiode array detector(PDA)

**MS Apparatus Type:** *Finnigan Surveyor* MSQ Plus (ION TRAP), lonisation mode ESI

### Abbreviations

| | |
|---|---|
| AcOEt | Acetic acid ethylester |
| AlCl₃ | Aluminium trichloride |
| CH₃CN | Acetonitrile |
| DCM | Dichloromethane |
| DMAP | 4-Dimethylamino pyridine |
| DMF | Dimethylformamide |
| DMSO | Dimethyl sulfoxide |
| eq. | Equivalent |
| iPAc | Isopropyl acetate |
| iPrOH | Isopropyl alcohol |
| LC-MS | High pressure liquid chromatography with MS detection |
| MgSO₄ | Manganese sulfate |
| MOM-Cl | Chloromethyl-methylether |
| mp | Melting point |
| NaHCO₃ | Sodium hydrogen carbonate |
| RT | Room temperature |
| Rₜ (01) | Retention time column / gradient 01 |
| Rₜ (02) | Retention time column / gradient 02 |
| TBME | Tertiary butyl methyl ether |
| tBuOK | Potassium tertiary butoxide |
| TFA | Trifluoroacetic acid |
| THF | Tetrahydrofuran |

### Example 1

This example illustrates the preparation of N-[4-(2,2-Dimethyl-propionylamino)-5-(3,4,5-trimethoxy-benzyl)-pyrimidin-2-yl]-2,2-dimethyl-propionamide **2** (R = C(CH₃)₃) (step A1).

A solution of trimethoprim (5 g, 17.24 mmol) in pivalic anhydride (8.74 mL, 43.10 mmol, 2.5 eq.) was heated during 2 h at 150 °C under argon. Hot AcOEt was added, and the organic layers were washed with aqueous NaHCO₃ 10%, water and brine. The org. layers were then dried over MgSO₄, filtered and evaporated. It was then recrystallized from TBME to give 3.02 g of compound **2** (R = C(CH₃)₃). ¹H-NMR (CDCl3, 400 MHz) δ: 8.35 (s, 1 H), 8.21 (br s, 1 H), 7.65 (br s, 1 H), 6.30 (s, 2 H), 3.86 (s, 2 H), 3.79 (s, 3 H), 3.77 (s, 6 H), 1.31 (s, 9 H), 1.12 (s, 9 H). mp: 130-133°C.

### Example 2

This example illustrates the preparation of N-[4-Isobutyrylamino-5-(3,4,5-trimethoxy-benzyl)-pyrimidin-2-yl]-isobutyramide **2** (R = CH(CH₃)₂) (step A1).

A solution of trimethoprim (50 g, 172.4 mmol) in isobutyric anhydride (100 g, 105 mL, 632 mmol, 3.6 eq.) was heated during 2 h at 150 °C under argon. The warm solution was poured into 1 L of cyclohexane from where it slowly crystallized. The product was filtered off and was washed thoroughly with cyclohexane (2x200 mL) to give 70 g of compound **2** (R = CH(CH₃)₂).

¹H-NMR (D₆-DMSO, 400 MHz) δ: 10.42 (s, 1H, NH); 10.15 (s, 1H, NH); 8.41 (s, 1H, pyrimidine); 6.41 (s, 2H, PhH); 3.81 (s, 2H, CH₂); 3.70 (s, 6H, 2xOCH₃); 3.59 (s, 3H, OCH₃); 2.72-2.85 (m, 2H, CH); 1.06 (d, 6H, J=6.6Hz, 2xCH₃), 1.01 (d, 6H, J=6.6Hz, 2xCH₃. mp: 153-154°C. Rₜ (02) = 1.65 minutes.

### Example 3

This example illustrates the preparation of N-[4-Isobutyrylamino-5-(3,4,5-trimethoxy-benzyl)-pyrimidin-2-yl]-isobutyramide **2** (R = CH(CH₃)₂) (step A1).

A solution of trimethoprim (50 g, 172.4 mmol) in isobutyric anhydride (62 g, 65.5 mL, 392 mmol, 2.3 eq.) was heated during 2 h at 150 °C under Ar and stirred with a mechanical stirrer. The solution was cooled to 130 °C and 200 ml toluene was added (clear solution), then 1000 ml TBME was slowly added (after 500 ml crystallization started) under vigorous stirring. The thick crystal cake was stirred for 1 hour at 100 °C external temperature. Then the slurry was cooled to RT and stirred for 2 hours. Finally the slurry was cooled to 10 °C and stirred for 2 hours. The crystals were filtered and washed with 3 times 90 ml TBME to remove residual isobutyric acid and anhydride. The crystals were dried at HV/70 °C for 8 hours to give 70 g of compound **2** (R = CH(CH₃)₂).

¹H-NMR (D₆-DMSO, 400 MHz) δ: 10.42 (s, 1H, NH); 10.15 (s, 1H, NH); 8.41 (s, 1H, pyrimidine); 6.41 (s, 2H, PhH); 3.81 (s, 2H, CH₂); 3.70 (s, 6H, 2xOCH₃); 3.59 (s, 3H, OCH₃); 2.72-2.85 (m, 2H, CH); 1.06 (d, 6H, J=6.6Hz, 2xCH₃), 1.01 (d, 6H, J=6.6Hz, 2xCH₃. mp: 153-154°C. Rₜ (02) = 1.65 minutes. See

### Example 4

This example illustrates the preparation of N-[4-(2,2-Dimethyl-propionylamino)-5-(2-formyl-3,4,5-trimethoxy-benzyl)-pyrimidin-2-yl]-2,2-dimethyl-propionamid **3** (R = C(CH₃)₃) (step B1).

To a solution of **2** (1 g, 2.18 mmol, R = C(CH₃)₃) in DCM (5 mL), dichloromethyl methyl ether (0.58 mL, 6.54 mmol) was added. The solution was cooled to -30 °C before slowly adding stannic chloride (0.285 mL, 2.18 mmol). The mixture was stirred at a temperature between -10 °C and -5 °C. At 0 °C, the reaction mixture was poured into a solution of 1-N K₃PO₄. The mixture (pH 7-8) was then vigorously stirred during 15 minutes, extracted twice with AcOEt. The organic layers were washed with water and brine, dried over MgSO₄, filtered and evaporated. The crude product was purified by flash chromatography on silica gel (AcOEt/Cyclohexane 7/3) to give 709 mg of compound **3** (R = C(CH₃)₃).

¹H-NMR (CDCl3, 400 MHz) δ: 10.23 (s, 1 H), 8.44 (s, 1 H), 8.12 (s, 1 H), 8.09 (s, 1 H), 6.59 (s, 1 H), 4.10 (s, 2 H), 3.96 (s, 3 H), 3.89 (s, 3 H), 3.85 (s, 3 H), 1.29 (s, 9 H), 1.27 (s, 9 H). mp: 124-126 °C.

### Example 5

This example illustrates the preparation of N-[5-(2-Formyl-3,4,5-trimethoxybenzyl)-4-isobutyrylamino-pyrimidin-2-yl]-isobutyramide **3** (R = CH(CH₃)₂) (step B1).

To a solution of **2** (70 g, 162 mmol, R = CH(CH₃)₂) in DCM (500 mL), dichloromethyl methyl ether (30 mL, 325 mmol) was added. The solution was cooled to -10 °C before slowly adding stannic chloride (35 mL, 300 mmol). The mixture was stirred at a temperature between -10 °C and -5 °C. At first a gummy precipitate was formed (mechanical stirrer required). After 1 h stirring at -5 °C this was transformed into a "homogeneous" suspension.

At 0°C, the reaction mixture is poured into a solution of 300 mL 1N K₃PO₄ and 200 mL 1 M Na/K-tartrate while cooling with an ice bath. The mixture (pH was adjusted with 4N NaOH solution to 7-8) was then stirred for 15 minutes until complete hydrolysis, and then extracted with DCM (300 mL) together with AcOEt (500 mL). The organic layer was washed with 0.1N HCl solution (2x200 mL) and brine (2x300 mL), dried over MgSO₄, filtered and evaporated. The product precipitated while concentration to about half of the initial volume, cyclohexane (200 mL) was added to further precipitate the product which was then filtered off to give 50 g of compound **3** (R = CH(CH₃)₂).

¹H-NMR (D₆-DMSO, 400 MHz) δ: (s, 1H, NH); 10.27 (s, 1H, NH); 10.17 (s, 1H, CHO); 8.00 (s, 1H, pyrimidine); 6.72 (s, 1H, PhH); 4.05 (s, 2H, CH₂); 3.90 (s, 3H, OCH₃); 3.85 (s, 3H, OCH₃); 3.77 (s, 3H, OCH₃); 2.75-2.85 (m, 2H, CH); 1.05 (d, 6H, J=6.6Hz, 2xCH₃), 1.01 (d, 6H, J=6.6Hz, 2xCH₃). mp: 162-163°C.
Rₜ (02) = 1.85 minutes.

### Example 6

This example illustrates the preparation of N-[5-(2-Formyl-3,4,5-trimethoxybenzyl)-4-isobutyrylamino-pyrimidin-2-yl]-isobutyramide **3** (R = CH(CH₃)₂) (step B1).

Dichloromethyl-methyl ether (4.3 mL, 46.4 mmol, 2 eq.) was dissolved in DCM (30 mL) and cooled to -15 °C to -20 °C in a reaction vessel with mechanical stirrer. To this solution stannic chloride (5 mL, 42.8 mmol, 1.8 eq.) was added within 15 minutes. The clear solution was stirred at -18°C for 30 minutes. Then a solution of **2** (10 g, 23.2 mmol, crystallized from toluene and TBME) in DCM (40 mL) was continuously added during 60 minutes, a yellow solid separated from the beginning and resulted in a thick slurry (green/yellow). Then the slurry was stirred at -15 °C for two hours, at -10 °C for one hour and 30 minutes at -5 °C. Then 40 mL DCM was added at -5 °C and the separated crystals at the top of the solvent layer were removed with vigorous stirring for 15 minutes. The thin slurry was transferred into a well-stirred mixture of 35 g Na₂CO₃ (with one crystal water) dissolved in 100 mL water and 35 mL DCM at 10 °C. The mixture was stirred for 15 minutes at RT and then transferred back to the reaction vessel to finish the workup continuing the stirring at RT. After vigorous stirring for 30 minutes at RT the layers were separated and the organic phase washed twice with a mixture of 30 mL saturated NaCl, 5 ml saturated Na₂CO₃ and 40 mL water (several shakings are necessary, the water phases should show a pH of 7 to 8). The milky water phases were washed with 50 mL DCM and 30 mL DCM separately.

The organic layers were dried over MgSO₄, filtered and evaporated. The crude white crystalline product was crystallized from DCM and TBME.

With 10.67 g crude material a slurry was made in 25 mL DCM at 44 °C under stirring for 30 minutes and 100 mL TBME were slowly added and the slurry stirred for 30 minutes at 44 °C and then cooled to RT for 6 hours under stirring. The crystals were filtered and washed with 40 mL TBME, dried at high vacuum/RT for 6 hours to give 9.6 g of compound **3** (R = CH(CH₃)₂).

¹H-NMR (D₆-DMSO, 400 MHz) δ: (s, 1H, NH); 10.27 (s, 1H, NH); 10.17 (s, 1H, CHO); 8.00 (s, 1H, pyrimidine); 6.72 (s, 1H, PhH); 4.05 (s, 2H, CH₂); 3.90 (s, 3H, OCH₃); 3.85 (s, 3H, OCH₃); 3.77 (s, 3H, OCH₃); 2.75-2.85 (m, 2H, CH); 1.05 (d, 6H, J=6.6Hz, 2xCH₃), 1.01 (d, 6H, J=6.6Hz, 2xCH₃). mp: 162-163°C.
Rₜ (02) = 1.85 minutes.

### Example 7

This example illustrates the preparation of N-[4-(2,2-Dimethyt-propionylamino)-5-(2-iodo-3,4,5-trimethoxy-benzyl)-pyrimidin-2-yl]-2,2-dimethyl-propionamide **5** (R = C(CH₃)₃) (step A2).

To a solution **4** (5 g, 9.2 mmol, R = C(CH₃)₃) in pivalic anhydride (4.1 mL, 20.24 mmol) was added pyridine (1.65 mL, 20.24 mmol). The mixture was heated at 120 °C during 12 h. HCl 0.25 N (25 mL) was added and the mixture was extracted twice with AcOEt. The organic layers were washed subsequently with water, NaHCO₃ 10 %, then water and brine, dried over MgSO₄, filtered and evaporated to dryness. The final compound was obtained by flash chromatography on silica gel (AcoEt/Cydohexane 1/1) to give 2.5 g of compound **5** (R = C(CH₃)₃). UV: 238 (282) nm.

### Example 8

This example illustrates the preparation of N-[4-(2,2-Dimethyl-propionylamino)-5-(2-formyl-3,4,5-trimethoxy-benzyl)-pyrimidin-2-yl]-2,2-dimethyl-propionamid **3** (R = C(CH₃)₃) (step B2).

**5** (1 g, 1.71 mmol, R = C(CH₃)₃) was dissolved in THF (10 mL) and the mixture was degassed with argon. Palladium tetrakis was then added (49.4 mg, 4 mol %). The mixture was heated to 70 °C and a slow stream of carbon monoxide gas was started. BU₃SnH (476 µL, 1.05 eq.) in 5 mL THF was slowly added over a period of 2.5 h. After 12 h at 70 °C, compound **3** (R = C(CH₃)₃) was isolated by flash chromatography. The analytical data were comparable to the compound of Example 4.

### Example 9

This example illustrates the preparation of N-[4-(2,2-Dimethyl-propionylamino)-5-(2-formyl-3-hydroxy-4,5-dimethoxy-benzyl)-pyrimidin-2-yl]-2,2-dimethyl-propionamide **6** (R = C(CH₃)₃) (step A3).

Under argon, **3** (2 g, 4.11 mmol, R = C(CH₃)₃) was dissolved in DCM (10 mL). AlCl₃ (823 mg, 6.17 mmol) was added to the mixture at 0 °C. Stirring was continued at RT during 10 minutes until complete dissolution of AlCl₃. Sodium iodide (616 mg, 4.11 mmol) was added, and after 30 minutes, 0.5 mL of acetonitrile was added. The reaction was checked by LCMS, and 0.5 mL acetonitrile was added to complete the reaction. The reaction mixture was then poured into 1 N K₃PO₄/DCM biphasic solution. The two phases were separated. The aqueous layers were extracted twice with AcOEt and the organic layers were washed with water and brine, then dried on MgSO₄, filtered and evaporated. Compound **6** (R = C(CH₃)₃) was obtained after purification on a column chromatography on silica gel eluting with AcOEt/cyclohexane 6/4 (1.2 g).

¹H-NMR (CDCl₃, 400 MHz) δ: 12.07 (*s,* 1 H), 9.81 (*s,* 1 H), 8.94 (*s,* 1 H), 8.26 (*s,* 1 H), 7.95 (*s,* 1 H), 6.33 (*s,* 1 H), 4.07 (*s,* 2 H), 3.84 (*s,* 3 H), 3.80 (*s,* 3 H), 1.22 (*s,* 9 H), 1.20 (*s,* 9 H). mp: 110-112°C.

### Example 10

This example illustrates the preparation of N-[5-(2-Formyl-3-hydroxy-4,5-dimethoxy-benzyl)-4-isobutyrylamino-pyrimidin-2-yl]-isobutyramide **6** (R = CH(CH₃)₂) (step A3).

Under argon, **3** (4 g, 8.7 mmol, 1 eq., R = CH(CH₃)₂) were dissolved in DCM (36 mL). Aluminium trichloride (3.48g, 26.1 mmol, 3 eq.) and sodium iodide (2 g, 13.3 mmol, 1.5 eq.) were added to the mixture at RT. The mixture was stirred for 20 minutes before acetonitrile (2.4 mL) was added and then warmed up to 40 °C. Stirring at 40 °C was continued for 3.5 h. The mixture was cooled to RT, diluted with 75 mL DCM and quenched by adding the reaction mixture to 30 mL ice-water, then 2.5 mL of concentrated HCl was slowly added, which helped to dissolve the yellow precipitate. The organic layer was separated and the aqueous layer extracted once more with DCM (75 mL). The combined organic layers were washed with brine (50 mL), twice with sodium bicarbonate solution made from 50 mL saturated sodium bi-carbonate (NaHCO₃) + 150 mL water (2x100 mL), 0.1N HCl solution (50 mL) and again brine (1x50 mL). The resulting yellowish solution was dried over MgSO₄ and concentrated. The oily residue was crystallized from ethyl acetate (6 mL) and dichloroethane (2.4 mL) by first warming to 50 °C, then cooling to 4 °C. After filtration the mother liquor was concentrated to halve and stored at 4 °C to give a second crop of crystals. In total 2.48 g of compound **6** (R = CH(CH₃)₂) were isolated.

¹H-NMR (CDCl₃, 400 MHz) δ: 11.95 (*br s,* 1H, PhOH); 9.9 (*s,* CHO, 1H), 8.04 (*s,* pyrimidine, 1H); 6.44 (*s*, ArH, 1H), 4.15 (*s,* CH₂, 2H), 3.93 (*s,* OCH₃, 3H); 3.90 (*s,* OCH₃, 3H); 2.7-2.8 (*m,* CH, 2H); 1.2-1.25 (*m,* CH₃, 12H).

### Example 11

This example illustrates the preparation of N-[5-(2-Formyl-3-hydroxy-4,5-dimethoxy-benzyl)-4-isobutyrylamino-pyrimidin-2-yl]-isobutyramide **6** (R = CH(CH₃)₂) (step A3).

Under argon, **3** (3 g, 6.54 mmol) was dissolved in DCM (28 mL) at RT (15 minutes). The solution was cooled to 0 °C during 30 minutes. AlCl₃ (2.07 g, 15.52 mmol, 2.3 eq.) was added to the cooled solution at once. The colour of the solution changed from yellow to dark yellow within 30 minutes and the AlCl₃ dissolved while stirring for 30 minutes at 0 °C. Then Nal (1.5 g, 10 mmol, 1.5 eq.) was added and the mixture warmed up to 30 °C. After 10 minutes stirring acetonitrile (1.6 mL) was slowly added. After 2 hours stirring at 30 °C additional 0.2 mL acetonitrile was added. After 4 hours stirring at 30 °C the reaction temperature was raised to 35 °C for 1 h, during this time crystals separated. The slurry was cooled to RT and then poured into a well stirred mixture of 20 mL DCM and 30 mL water containing 2 mL concentrated HCl (cooled to 10 °C). After stirring for 10 minutes the clear yellow mixture was poured back to the reaction vessel and the stirring was continued until all the residues were dissolved (about 30 minutes). The organic phase was separated and washed with 25 mL of a mixture of 10 mL 1-N HCl and 15 mL water and 25 mL of a mixture of 10 ml saturated NaCl and 15 ml water, the water layers were extracted with 2 times 20 mL DCM. The combined organic solution was dried with MgSO₄, filtered and evaporated. The yellow crystalline residue (2.62 g) was crystallized from DCM/TBME to give 2.48 g of compound **6** (R = CH(CH₃)₂).

¹H-NMR (CDCl₃, 400 MHz) δ: 11.95 (*br s*, 1H, PhOH); 9.9 (*s*, CHO, 1H), 8.04 (s, pyrimidine, 1H); 6.44 (*s*, ArH, 1H), 4.15 (*s,* CH₂, 2H), 3.93 (*s,* OCH₃, 3H); 3.90 (*s*, OCH₃, 3H); 2.7-2.8 (*m,* CH, 2H); 1.2-1.25 (*m,* CH₃, 12H).

### Example 12

This example illustrates the preparation of N-[4-(2,2-Dimethyl-propionylamino)-5-(2-formyl-4,5-dimethoxy-3-methoxymethoxy-benzyl)-pyrimidin-2-yl]-2,2-dimethyl-propionamide **7** (R = C(CH₃)₃, R' = methoxymethyl) (step A4).

Under argon, **6** (200 mg, 0.424 mmol, R = C(CH₃)₃) was dissolved in DCM (2 mL). triethylamine (59.6 µL, 1 eq.) was added to the mixture at 0 °C. MOM-Cl (32.2 µL, 1 eq.) was slowly added. After stirring for 1 h 1 equivalent of triethylamine and 1 equivalent of MOM-Cl were added to complete the reaction.

0.25 N HCl was added to the reaction mixture and the mixture was extracted with AcOEt. The organic layers were washed with water and brine, then dried over MgSO₄, filtered and evaporated. Compound **7** (R = C(CH₃)₃, R' = methoxymethyl) was used directly in the next reaction. UV: 234 (283) nm.

### Example 13

This example illustrates the preparation of N-[4-(2,2-Dimethyl-propionylamino)-5-(2-formyl-4,5-dimethoxy-3-allyloxy-benzyl)-pyrimidin-2-yl]-2,2-dimethyl-propionamide **7** (R = CH(CH₃)₂, R' = allyl) (step A4).

A solution of **6** (166.9 g, 0.35 mol, R = CH(CH₃)₂, R' = allyl) in DMF (800 mL) was cooled to 0 °C and tBuOK (47 g, 0.42 mol) was added. To this solution allyl bromide (41 mL, 0.49 mol) was slowly added and stirred for 3 hours at 0 °C and 16 hours at RT. The reaction mixture was filtered and the solvent distilled off at reduced pressure (20 mbar) and 60 °C. The residue was diluted with DCM (850 mL) and the organic phase was washed with HCl 1 N (250 mL), saturated sodium bicarbonate (500 mL) and water (500 mL), then dried over MgSO₄, filtered and evaporated to give 175 g of crystalline 7 (R = CH(CH₃)₂, R' = allyl).

¹H-NMR (CDCl₃, 400 MHz) δ: 10.34. (*s,* 1 H), 9.37 (broad, NH), 8.64 (broad, NH), 8.32 (*s,* 1 H), 6.53 (*s,* 1 H), 6.02 (*m,* 1 H), 5.31 (*m,* 2 H), 4.70 (*m,* 2 H), 4.12 (*s,* 2 H), 3.87 (*s,* 3 H), 3.84 (*s,* 3 H), 2.91 (broad, 1H), 2.67 (*m,* 1 H), 1.22 (*d*, *J* = 7.2 Hz, 6H), 1.17 (*d*, *J*= 7.2 Hz, 6H)

### Example 14

This example illustrates the preparation of N-{5-[2-(3-Cyclopropyl-3-oxo-propenyl)-4,5-dimethoxy-3-methoxymethoxy-benzyl]-4-(2,2-dimethyl-propionylamino)-pyrimidin-2-yl}-2,2-dimethyl-propionamide **9** (R = C(CH₃)₃, R' = methoxymethyl) (step B4).

Under argon, **7** (140 mg, 0.27 mmol, R = C(CH₃)₃, R' = methoxymethyl) was dissolved in THF (1 mL). In a second flask, the enolate of acetylcyclopropane **8** was prepared (28 µL, 1.1 eq.), *t*BuOK (34 mg, 1.1 eq.) in 1 mL THF and after 3 minutes the enolate was added dropwise to the solution of **7** (R = C(CH₃)₃, R' = methoxymethyl) at RT. After 5 minutes, another equivalent of the enolate of **8** was added to complete the reaction. After 1 h, 0.25 N HCl was added to the reaction mixture and the mixture was extracted twice with AcOEt. The organic layers were washed with water and brine, then dried over MgSO₄, filtered and evaporated. The crude material was purified by flash chromatography on silica gel (AcOEt/cyclohexane 5/5 → AcOEt/cyclohexane 7/3) to give 0.133 g of compound **9** (R = C(CH₃)₃, R' = methoxymethyl).

¹H-NMR (CDCl₃, 400 MHz) δ: 8.22 (*s,* 1 H), 8.15 (*s*, 1 H), 8.08 (*s*, 1 H), 7.44 (*d, J*= 16.4 Hz, 1 H), 6.78 (*d, J*= 16.4 Hz, 1 H), 6.51 (*s*, 1 H), 5.06 (*s*, 2 H), 3.83 (*s,* 2 H), 3.77 (*s,* 3 H), 3.76 (*s,* 3 H), 3.43 (*s,* 3 H), 1.98-2.04 (*m,* 1 H), 1.22 (*s*, 9 H), 1.19 (*s*, 9 H), 1.01 (*quint, J*= 3.6 Hz, 2 H), 0.82-0.86 (*m,* 2 H).

### Example 15

This example illustrates the preparation of N-{5-[2-(3-Cyclopropyl-3-oxo-propenyl)-4,5-dimethoxy-3-allyloxy-benzyl]-4-(2,2-dimethyl-propionylamino)-pyrimidin-2-yl}-2,2-dimethyl-propionamide **9** (R = CH(CH₃)₂, R' = allyl) (step B4).

Under argon, **7** (424 mg, 0.877 mmol, R = CH(CH₃)₂, R' = allyl) was dissolved in THF/DMF (6 mL, 2:1). In a second flask, the enolate of acetylcyclopropane **8** was prepared (109 µL, 1.1 eq.), *t*BuOK (131 mg, 1.1 eq.) in 2 mL THF and after 3 minutes the enolate was added dropwise to the solution of **7** (R = CH(CH₃)₂, R' = allyl) at RT. After 5 minutes, another equivalent of the enolate of **8** was added to complete the reaction. After 3 days, the reaction mixture was diluted with DCM and the mixture was washed three times with sodium hydrogen carbonate 5% then dried over MgSO₄, filtered and evaporated. The crude material was purified by flash chromatography on silica gel (AcOEt/toluene 1/1) to give 0.340 g of compound **9** (R = CH(CH₃)₃, R' = allyl). The compound was crystallized from TBME to give 0.210 g crystals.

¹H-NMR (CDCl₃, 400 MHz) δ: 8.80 (broad, 2 NH), 8.08 (*s*, 1 H), 7.45 (*d, J*= 16.2 Hz, 1 H), 6.94 (*d, J*= 16.2 Hz, 1 H), 6.61 (*s*, 1 H), 6.06 (*m,* 1H), 5.30 (*m,* 2 H), 4.53 (*m,* 2 H), 3.91 (*s*, 2 H), 3.87 (*s*, 3 H), 3.86 (*s*, 3 H), 3.13 (broad, 1H), 2.27 (*m,* 1 H), 2.04 (*m,* 1 H), 1.22 (*d, J*= 2.0 Hz, 6 H), 1.20 (*d, J*= 2.5 Hz, 6 H), 1.06 (*m,* 2H), 0.91 (*m,* 2 H).

### Example 16

This example illustrates the preparation of N-{5-[2-(3-Cyclopropyl-3-hydroxypropenyl)-4,5-dimethoxy-3-methoxymethoxy-benzyl]-4-(2,2-dimethyl-propionylamino)-pyrimidin-2-yl}-2,2-dimethyl-propionamide **10** (R = C(CH₃)₃, R' = methoxymethyl) (step C4).

Under argon, **9** (418 mg, 0.718 mmol, R = C(CH₃)₃, R' = methoxymethyl) was dissolved in iPrOH (5 mL). NaBH₄ (54 mg, 1.436 mmol) was added at RT. After 2h, the reaction was stopped. Water was added to the reaction mixture and it was extracted twice with AcOEt. The organic layers were washed with water and brine, then dried over MgSO₄, filtered and evaporated. The crude material was purified by flash chromatography on silica gel (AcOEt/cyclohexane 8/2) to give 0.29 g of compound **10** (R = C(CH₃)₃, R' = methoxymethyl).

¹H-NMR (CDCl₃, 400 MHz) δ: 8.72 (*s,* 1 H), 8.15 (*s*, 1 H), 7.92 (*s,* 1 H), 6.46 (*d,* J= 16.4 Hz, 1 H), 6.34 (*s,* 1 H), 6.05 (*dd, J*₁ = 16.4 Hz, *J*₂= 6.2 Hz, 1 H), 5.04 (*s*, 2 H), 3.83 (*s,* 2 H), 3.78 (*s*, 5 H), 3.70 (*s*, 3 H), 3.57 (*m,* 1 H), 3.51 (*s*, 3 H), 1.29 (*s*, 9 H), 1.05 (*s,* 9 H), 037-0.56 (*m,* 2 H), 0.20-0.26 (*m,* 2 H).

### Example 17

This example illustrates the preparation of N-{5-[2-(3-Cyclopropyl-3-hydroxypropenyl)-4,5-dimethoxy-3-methoxymethoxy-benzyl]-4-(2,2-dimethyl-propionylamino)-pyrimidin-2-yl}-2,2-dimethyl-propionamide **10** (R = CH(CH₃)₂, R' = allyl) (step C4).

Under argon, **9** (100 mg, 0.181 mmol, R = CH(CH₃)₂, R' = allyl) was dissolved in iPrOH (2 mL). NaBH₄ (13 mg, 0.363 mmol) was added at RT. After 2 hours at RT the reaction was kept at 4 °C for 16 hours. Water was added to the reaction mixture and it was extracted twice with AcOEt. The organic layers were dried over MgSO₄, filtered and evaporated to give 0.11 g of compound **10** (R = CH(CH₃)₂, R' = allyl). The crude material was used directly without purification for the next reaction step.

LC-MS (Gradient 01): Rₜ = 8.43 [M+H]⁺ = 553

### Example 18

This example illustrates the preparation of N-[5-(2-Cyclopropyl-7,8-dimethoxy-2H-chromen-5-ylmethyl)-4-(2,2-dimethyl-propionylamino)-pyrimidin-2-yl]-2,2-dimethyl-propionamide **11** (R = C(CH₃)₃) (step D4).

Under argon, **10** (180 mg, 0.308 mmol, R = C(CH₃)₃, R' = methoxymethyl) was dissolved in DCM (2 mL). TFA (24 µL, 0.308 mmol) was added and after 30 minutes at RT 2 equivalent of TFA were added. After 5 minutes NaHCO₃ 10 % (10 mL) was added, and the mixture was extracted with AcOEt. The organic layers were washed with water and brine, then dried over MgSO₄, filtered and evaporated. The crude material was purified by flash chromatography on silica gel (AcOEt/cyclohexane 4/6 → AcOEt/cyclohexane 5/5) to give 0.1 g of compound **11** (R = C(CH₃)₃).

¹H-NMR (CDCl₃, 400 MHz) δ: 8.19 (*s,* 1 H), 8.12 (*s,* 1 H), 8.00 (*s,* 1 H), 6.38 (*d, J*= 10 Hz, 1 H), 6.16 (*s,* 1 H), 5.67 (dd, *J*₁ = 10.2 Hz, *J*₂= 3.4 Hz, 1 H), 4.19 (*dd, J*₁ = 8.4 Hz, *J*₂= 3.4 Hz, 1 H), 3.87 (*s,* 2 H), 3.80 (*s,* 2 H), 3.75 (*s,* 3 H), 1.30 (*s,* 9 H), 1.21 (*s,* 9 H), 0.43-0.61 (*m,* 3 H), 0.26-0.34 (*m,* 2 H).

### Example 19

This example illustrates the preparation of N-[5-(2-Cyclopropyl-7,8-dimethoxy-2H-chromen-5-ylmethyl)-4-(2,2-dimethyl-propionylamino)-pyrimidin-2-yl]-2,2-dimethyl-propionamide **11** (R = CH(CH₃)₂) (step D4).

Under Argon, **10** (110 mg, 0.18 mmol, R = CH(CH₃)₂, R' = allyl) was dissolved in acetonitrile (2 mL). Tetrakis(triphenylphosphine)palladium (46 mg, washed with DMSO, EtOH, diethyl ether) and ammonium formiate (57 mg) was added and the slurry heated to 70 °C for 2 hours. After cooling to RT the reaction mixture was diluted with EtOAc (30 mL) and washed with water. The organic layer was washed with water and brine, then dried over MgSO₄, filtered and evaporated to give an oil which was used in the next reaction step without purification.

LC-MS (Gradient 01): Rₜ = 7.33 [M+H]⁺ = 495

### Example 20

This example illustrates the preparation of N-[5-(2-Acetyl-3,4,6-trimethoxy-benzyl)-4-(2,2-dimethyl-propionylamino)-pyrimidin-2-yl]-2,2-dimethyl-propionamide **12** (R = C(CH₃)₃) (step A5).

Under argon, **2** (2 g, 4.37 mmol, R = C(CH₃)₃) was dissolved in DCM (5 mL). Acetic anhydride was added (2.06 mL, 5 eq.) followed by the addition of SnCl₄ (2.55 mL, 5 eq.) at 0 °C. Stirring was continued at RT, after 4 h the reaction was treated with NaHCO₃ 10 % until pH = 9 and the mixture was extracted twice with AcOEt. The organic layers were washed with water and brine, then dried over MgSO₄, filtered and evaporated. The crude material was purified by flash chromatography on silica gel (AcOEt/cyclohexane 6/4 to 7/3) to give 1.4 g of compound **12** (R = C(CH₃)₃).

¹H-NMR (CDCl₃, 400 MHz) δ: 8.28 (*s,* 1 H), 8.22 (*s*, 1 H), 8.14 (*s*, 1 H), 6.34 (*s*, 1 H), 3.89 (*s*, 3 H), 3.81 (*s*, 3 H), 3.80 (*s*, 2 H), 3.75 (*s*, 3 H), 2.41 (*s*, 3 H), 1.30 (*s,* 9 H), 1.18 (*s*, 9 H).
mp: 94-98 °C.

### Example 21

This example illustrates the preparation of N-[5-(2-Acetyl-3-hydroxy-4,5-dimethoxy-benzyl)-4-(2,2-dimethyl-propionylamino)-pyrimidin-2-yl]-2,2-dimethyl-propionamide **13** (R = C(CH₃)₃) (step B5).

Under argon, **12** (2.34 g, 4.68 mmol, R = C(CH₃)₃) was dissolved in DCM (20 mL). At 0 °C, AlCl₃ (1.872 g, 14.04 mmol) was added and stirring was continued until complete dissolution. Sodium iodide was then added (702 mg, 4.68 mmol), followed 30 minutes later by 3 mL of CH₃CN. The reaction was stirred until complete disappearance of starting material (LCMS). AcOEt and HCl 0.1 N were added to the reaction mixture and the mixture was extracted twice with AcOEt. The organic layers were washed with water and brine, then dried over MgSO₄, filtered and evaporated. The crude material was purified by flash chromatography on silica gel (AcOEt/cyclohexane 8/2) to give 1.5 g of compound 13 (R = C(CH₃)₃). ¹H-NMR (CDCl₃, 400 MHz) δ: 8.42 (*s,* 2 H), 8.22 (*s,* 1 H), 6.20 (*s,* 1 H), 3.92 (*s*, 2 H), 3.84 (*s,* 3H), 3.77 (*s*, 3H), 2.50 (*s*, 3H), 1.29 (*s*, 9H), 1.19 (*s,* 9 H).

### Example 22

This example illustrates the preparation of N-[5-(2-Cyclopropyl-7,8-dimethoxy-4-oxo-chroman-5-ylmethyl)-4-(2,2-dimethyl-propionylamino)-pyrimidin-2-yl]-2,2-dimethyl-propionamide **15** (R = C(CH₃)₃) (step C5).

**13** (432 mg, 0.889 mmol, R = C(CH₃)₃) was dissolved in CH₃CN (10 mL). Pyrrolidine (81 µL, 0.978 mmol) and cyclopropylcarboxaldehyde **14** (73 µL, 0.978 mmol) were added and the reaction was stirred overnight at RT. AcOEt and NaHCO₃ 10 % were added. The organic layers were washed with HCl 0.5 N, water and brine, then dried over MgSO₄, filtered and evaporated to dryness. The crude material was purified by flash chromatography on silica gel (AcOEt/cyclohexane 7/3) to give 0.24 g of compound **15** (R = C(CH₃)₃).

¹H-NMR (CDCl₃, 400 MHz) δ: 8.70 (*br s*, 1 H), 8.37 (*br s,* 1 H), 8.04 (*s,* 1 H), 6.51 (*s*, 1 H), AB system (δ_{A} 4.20, δ_{B} 4.07, *J_{AB}*= 15.6 Hz, 2 H), 3.91 (*s,* 3 H), 3.85 (*s,* 3 H), 3.64-3.70 (*m,* 1 H), 2.61-2.76 (*m,* 2 H), 1.34 (*s,* 9 H), 1.29 (*s,* 9 H), 0.59-0.74 (*m,* 2 H), 0.50-0.57 (*m,* 1 H), 0.28-0.38 (*m,* 1 H).

### Example 23

This example illustrates the preparation of N-{5-[2-(3-Cyclopropyl-acryloyl)-3,4,5-trimethoxy-benzyl]-4-(2,2-dimethyl-propionylamino)-pyrimidin-2-yl}-2,2-dimethyl-propionamide **17** (R = C(CH₃)₃) (step A6).

Under argon, **2** (0.5 g, 1.09 mmol, R = C(CH₃)₃) was dissolved in DCM (5 mL) and compound **16** was added (0.71 g, 5 eq.) followed by the addition of SnCl₄ (0.63 mL, 5 eq.) at 0 °C. Stirring was continued at RT, after 4 h the reaction was treated with NaHCO₃ 10 % until pH = 9 and the mixture was extracted twice with AcOEt. The organic layers were washed with water and brine, then dried over MgSO₄, filtered and evaporated. The crude material was purified by flash chromatography on silica gel (AcOEt/cyclohexane 6/4 to 7/3) to give compound **17** (R = C(CH₃)₃).

¹H-NMR (CDCl₃, 400 MHz) δ: 0.58-0.61 (*m,* 2 H), 0.93-0.98 (*m,* 2 H), 1.16 (*s,* 9 H), 1.28 (*s,* 9 H), 1.51-1.60 (*m,* 1 H), 3.71 (*s,* 2 H), 3.74 (*s,* 3 H), 3.78 (*s,* 6 H), 6.02 *(dd, J₁=* 15.2 Hz, *J₂=* 10 Hz, 1 H), 6.35 (*s,* 1 H), 6.38 (*d, J*= 15.2 Hz, 1 H), 8.15 (*br s,* 1 H), 8.28 (*s,* 1 H), 8.35 (*br s,* 1H).

### Example 24

This example illustrates the preparation of N-{5-[2-(3-Cyclopropyl-acryloyl)-3,4,5-trimethoxy-benzyl]-4-(2,2-dimethyl-propionylamino)-pyrimidin-2-yl}-2,2-dimethyl-propionamide **17** (R = C(CH₃)₃) (step B6).

**12** (500 mg, 1 mmol, R = C(CH₃)₃) was dissolved in THF (10 mL), tBuOK (168.3 mg, 1.5 mmol) was added and the mixture stirred 10 minutes at RT. Cyclopropylcarboxaldehyde **14** (82 µL, 1.1 mmol) was added and the reaction was stirred for 30 minutes at RT. After addition of AcOEt and HCl 0.1 N, the organic layers were washed with water and brine, then dried over MgSO₄, filtered and evaporated to dryness. The crude material was purified by flash chromatography on silica gel (AcOEt/cyclohexane 6/4) to give 0.45 g of the compound **17** (R = C(CH₃)₃) having the same NMR given in Example 23.

### Example 25

This example illustrates the preparation of N-{5-[2-(3-Cyclopropyl-3-iodo-propionyl)-3-hydroxy-4,5-dimethoxy-benzyl]-4-(2,2-dimethyl-propionylamino)-pyrimidin-2-yl}-2,2-dimethyl-propionamide **18** (R = C(CH₃)₃) (step C6).

**17** (152 mg, 0.275 mmol, R = C(CH₃)₃) was dissolved in DCM (2 mL). Sodium iodide (103 mg, 0.6875 mmol) was added and the mixture was stirred for 5 minutes before adding aluminium trichloride (55 mg, 0.413 mmol). Stirring was continued for 30 minutes at RT, then 0.5 mL of CH₃CN were added. After 2h AcOEt was added and the organic layer was washed with water and brine, then dried over MgSO₄, filtered and evaporated to dryness. The crude material was purified by flash chromatography on silica gel (AcOEt/cyclohexane 7/3) to give 0.11 g of compound **18** (R = C(CH₃)₃).

¹H-NMR (CDCl₃, 400 MHz) δ: 8.6 (*br s,* 1 H), 8.46 (*br s,* 1 H), 8.30 (*s,* 1 H), 6.70 (*td, J*= 6.4 Hz, 1 H), 6.49 (*d, J*= 16 Hz, 1 H), 6.21 (*s*, 1 H), 3.86 (*s*, 2 H), 3.85 (*s,* 3 H), 3.78 (*s,* 3 H), 3.17 (t, *J*=7 Hz, 2 H), 2.33 (*quint, J*= 6.9 Hz, 2 H), 1.95 (*quint, J*= 7.1 Hz, 2 H), 1.30 (*s,* 9H), 1.18 (*s,* 9 H).

### Example 26

This example illustrates the preparation of N-[5-(2-Cyclopropyl-7,8-dimethoxy-4-oxo-chroman-5-ylmethyl)-4-(2,2-dimethyl-propionylamino)-pyrimidin-2-yl]-2,2-dimethyl-propionamide **15** (R = C(CH₃)₃) (step D6). **18** (300 mg, 0.45 mmol, R = C(CH₃)₃) was dissolved in DMF (5 mL). K₂CO₃ (124 mg, 0.9 mmol) was added and the mixture was stirred 2 h at RT. The mixture was then stirred during 3 h at 50 °C. After addition of AcOEt the organic layer was washed with water and brine, then dried over MgSO₄, filtered and evaporated to dryness. The crude material was purified by flash chromatography on silica gel (AcOEt/cyclohexane 7/3) to give 0.075 g of compound **15** (R = C(CH₃)₃) having the same NMR given in Example 22.

### Example 27

This example illustrates the preparation of N-[5-(2-Cyclopropyl-4-hydroxy-7,8-dimethoxy-chroman-5-ylmethyl)-4-(2,2-dimethyl-propionylamino)-pyrimidin-2-yl]-2,2-dimethyl-propionamide **19** (R = C(CH₃)₃) (step A7).

NaBH₄ (11.6 mg, 1.5 eq.) was added at 0 °C to a solution of **15** (110 mg, 0.205 mmol, R = C(CH₃)₃) in 2 mL iPrOH. The mixture was stirred 1 h at RT. Water was added and the mixture was extracted twice with AcOEt. The organic layers were washed with brine, dried over MgSO₄, filtered and evaporated to dryness. The crude material was purified by flash chromatography on silica gel (AcOEt/cyclohexane 8/2 to AcOEt 100%) to give 0.073 g of compound 19 (R = C(CH₃)₃).

¹H-NMR (CDCl₃, 400 MHz) δ: 8.44 (*br s,* 1H), 8.39 (*br s,* 1 H), 8.11 (*s,* 1 H), 6.20 (*s,* 1 H), 4.92 (*br s,* 1 H), AB system (δ_{A} 4.02, δ_{B} 3.91, *J_{AB}=* 16 Hz, 2 H), 3.80 (*s,* 3 H), 3.72 (*s,* 3 H), 3.40 (*td*, *J₁*= 8.6 Hz, *J₂*=2.4 Hz, 1 H), 2.35-2.41 (*m,* 1 H), 2.1-2.19 (*m,* 1 H), 1.24 (*s,* 9 H), 1.13 (*s*, 9 H), 0.54-0.61 (*m,* 2 H), 0.43-0.48 (*m,* 1 H), 0.27-0.32 (*m,* 1 H).

### Example 28

This example illustrates the preparation of N-[5-(2-Cyclopropyl-7,8-dimethoxy-2H-chromen-5-ylmethyl)-4-(2,2-dimethyl-propionylamino)-pyrimidin-2-yl]-2,2-dimethyl-propionamide **11** (R = C(CH₃)₃) (step B7).

TFA (36 µL, 5 eq.) was added to a solution of **19** (50 mg, 92.6 µmol, R = C(CH₃)₃) in toluene (2 mL). The mixture was heated at 80 °C during 12 h. The mixture was then washed with NaHCO₃ 10 %, extracted twice with AcOEt. The organic layers were washed with water, brine then dried over MgSO₄, filtered and evaporated to dryness to give crude **11** (R = C(CH₃)₃) which was further used without purification.

### Example 29

This example illustrates the preparation of 5-(2-Cyclopropyl-7, 8-dimethoxy-2H-chromen-5-ylmethyl)-pyrimidine-2, 4-diamine I (step A8).

Under argon, **11** (100 mg, 0.192 mmol, R = C(CH₃)₃) was dissolved in methanol (1 mL). Sodium methoxide (42 mg, 0.766 mmol) was added. The reaction mixture was heated at 50 °C. After 10 h, methanol was removed under reduced pressure and the yellow solid was triturated in water. The white precipitate was filtered off, washed with water and dried under high vacuum to give 0.062 g of compound I.

¹H-NMR (CDCl₃, 400 MHz) δ: 7.06 (s, 1 H), 6.45 (d, J= 10.4 Hz, 1 H), 6.42 (s, 1 H), 6.22 (br s, 2 H), 5.70-5.73 (m, 3 H), 4.25 (dd, J₁ = 8.2 Hz, J₂= 2.6 Hz, 1 H), 3.72 (s, 3 H), 3.70 (s, 3 H), 3.52 (br s, 2 H), 1.09-1.18 (m, 1 H), 1.21 (s, 9 H), 0.29-0.53 (m, 4 H).

### Example 30

This example illustrates the preparation of 5-(2-Cyclopropyl-7, 8-dimethoxy-2H-chromen-5-ylmethyl)-pyrimidine-2, 4-diamine I (step A8)

Under argon, **11** (crude material from Example 19, R = CH(CH₃)₂) was dissolved in THF (2 mL). Sodium hydroxide (0.6 mL, 4 N) was added and the reaction mixture heated at 58 °C for 2 hours. The reaction mixture was diluted with EtOAc/15% iPrOH and the organic layer was 2 times washed with brine, then dried over MgSO₄, filtered and evaporated to dryness to give crude I oil. The analytical data are compatible with the data from Example 29.

LC-MS (Gradient 01): Rₜ = 4.80 [M+H]⁺ = 355

## Claims

1. Process for manufacturing a compound of formula I 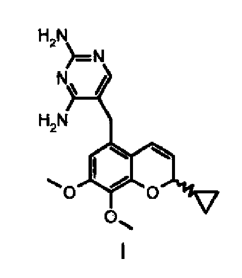 wherein a compound of formula 11 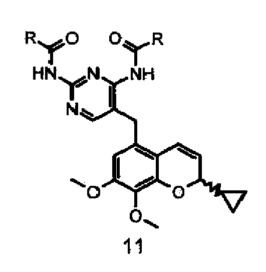 wherein R represents -C(CH₃)₃ or -CH(CH₃)₂,
is deprotected to obtain the target compound I.

2. The process according to claim 1, wherein the compound of formula **11** is deprotected in a mixture of solvents consisting of tetrahydrofuran or methyl alcohol, and water with a strong alkali base in a temperature range of 40°C to 80°C.

3. The process according to claim 1 or 2, wherein the compound of formula **11** is obtained by performing a palladium complex or/and acid catalyzed cyclisation of a compound of formula **10** 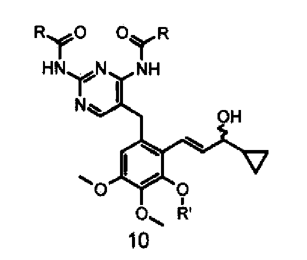 wherein R has the meaning given in claim 1 and R' represents methoxymethyl or allyl;
the compound of formula **10** is obtained by reducing a compound of formula **9** 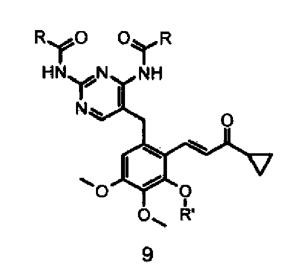 wherein R and R' have the meaning given in formula **10** above;
the compound of formula **9** is obtained by reacting a compound of formula **7** 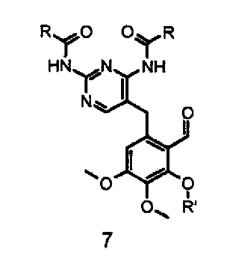 with a ketone of formula **8** 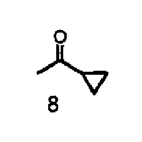 wherein R and R' have the meaning given in formula **10** above;
the compound of formula **7** is obtained by protecting the phenol group of a compound of formula **6** 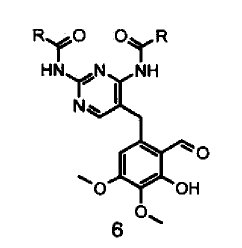 wherein R has the meaning given in formula **10** above.

4. The process according to claim 3, wherein the compound of formula **6** is
obtained by selective demethylation of a compound of formula **3** 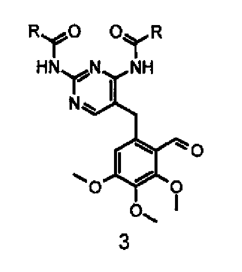 wherein R has the meaning given in Claim 1.

5. The process according to claim 4, wherein the compound of formula **3** is obtained by formylating a compound of formula **2,** 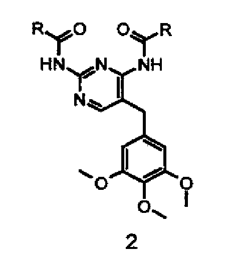 wherein R has the meaning given in Claim 1;
the compound of formula **2** is obtained by selectively protecting the diamino pyrimidine substituent of a compound of the formula **1**

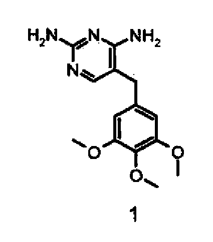

6. The process according to claim 4, wherein a compound of formula **3** is obtained
by by carbonylation of a compound of the formula **5** 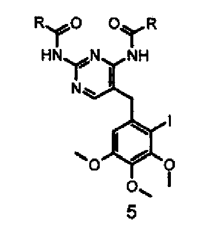 wherein R has the meaning given in Claim 1;
the compound of formula **5** is obtained by protecting the diamino pyrimidine group of a compound of formula **4**

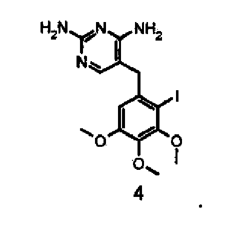

7. The process according to claim 1 or 2, wherein the compound of formula **11** is
obtained by water elimination of a compound of formula **19** 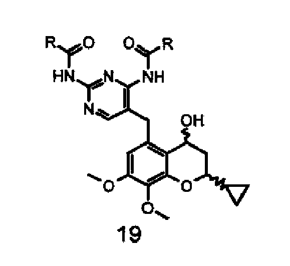 with an acid at room temperature up to 100°C, wherein R has the meaning given in Claim 1,

8. The process according to claim 7, wherein the compound of formula **19** is
obtained by reducing a compound of the formula **15** 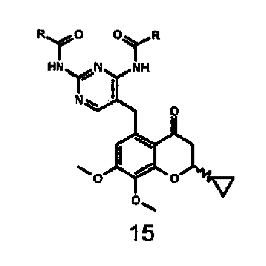 wherein R has the meaning given in claim 1.

9. The process according to claim 8, wherein the compound of formula **15** is
obtained by reacting a compound of the formula **13** 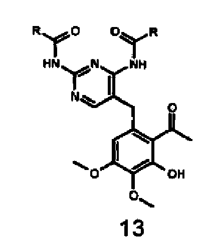 wherein R has the meaning given in claim 1 with a compound of the formula **14** 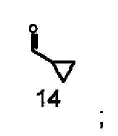 the compound of the formula **13** is obtained by selectively demethylating a compound of the formula **12** 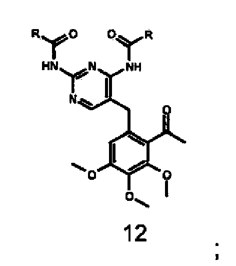 wherein R has the meaning given in formula **13** above;
the compound of the formula **12** is obtained by acylating a compound of the formula **2** 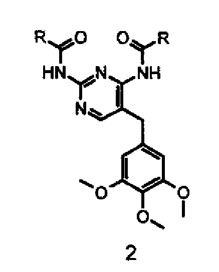 wherein R has the meaning given in formula **13** above.

10. The process according to claim 8 wherein the compound of formula **15** is
obtained by cyclisation of a compound of the formula **18** 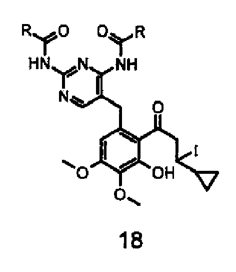 wherein R has the meaning given in claim 1;
the compound of the formula **18** is obtained by selective demethylation of a compound of the formula **17** 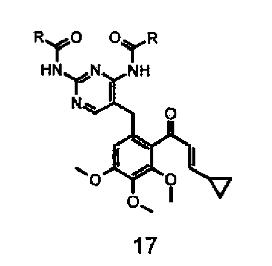 by treatment with sodium iodide, wherein R has the meaning given in formula **18** above.

11. The process according to claim 10, wherein the compound of formula **17** is obtained by acylation of a compound of the formula **2** 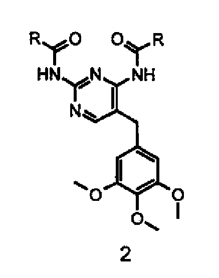 wherein R has the meaning given in claim 1, with a compound of formula **16**

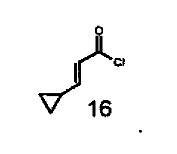

12. The process according to claim 10, wherein the compound of formula **17** is obtained by the reaction of a compound of the formula **12** 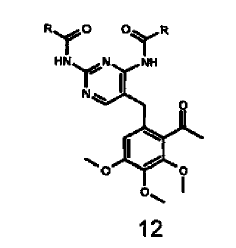 wherein R has the meaning given in claim 1, with a compound of the formula **14**

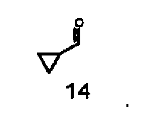

13. The process for manufacturing the compound of formula I according to claim 1 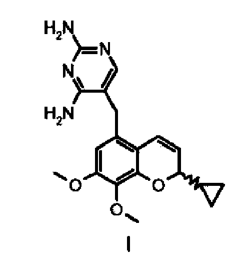 starting with a novel common intermediate of formula **6** 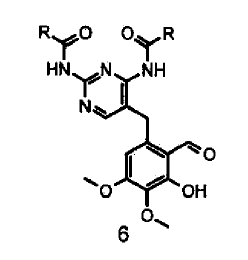 wherein
R represents -C(CH₃)₃ or -CH(CH₃)₂,
protecting the phenol group of the compound of formula **6** above to obtain a compound of formula **7** 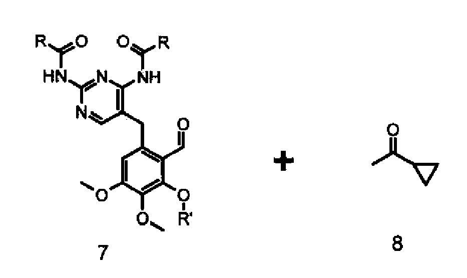 wherein R has the meaning given in formula **6** above and R' represents methoxymethyl or allyl
reacting the compound of formula **7** with the ketone of formula **8** to obtain the compound of formula **9** 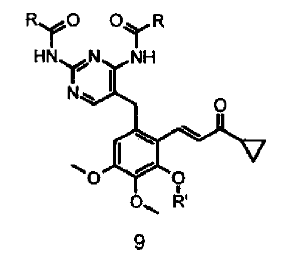 wherein R and R' have the meaning given in formula **7** above,
reducing the compound of formula **9** to the compound of formula **10** 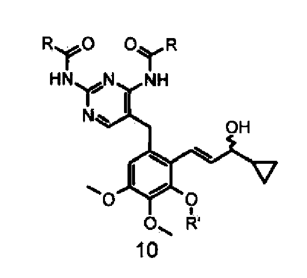 wherein R and R' has the meaning given in formula **7** above,
performing an palladium complex or / and acid catalyzed cyclisation to obtain the compound of formula **11** 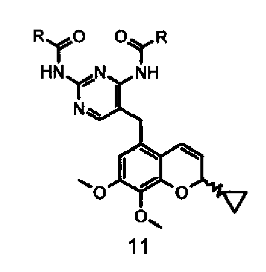 wherein R has the meaning given in formula **6** above,
and deprotecting the compound of formula **11** in a manner known per se to obtain the target compound I.

14. The process according to claim 13 wherein the compound of formula **11** 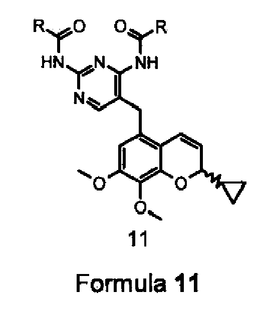 wherein
R represents -C(CH₃)₃ or -CH(CH₃)₂,
is deprotected in a mixture of solvents consisting of tetrahydrofuran or methyl alcohol, and water with a strong alkali base in a temperature range of 40°C to 80°C to obtain the target compound of formula I.

15. Intermediate compound selected from the group consisting of
N-[4-(2,2-Dimethyl-propionylamino)-5-(3,4,5-trimethoxy-benzyl)-pyrimidin-2-yl]-2,2-dimethyl-propionamide formula **2** (R = C(CH₃)₃)
N-[4-Isobutyrylamino-5-(3,4,5-trimethoxy-benzyl)-pyrimidin-2-yl]-isobutyramide formula 2 (R = CH(CH₃)₂)
N-[4-(2,2-Dimethyl-propionylamino)-5-(2-formyl-3,4,5-trimethoxy-benzyl)-pyrimidin-2-yl]-2,2-dimethyl-propionamid formula **3** (R = C(CH₃)₃)
N-[5-(2-Formyl-3,4,5-trimethoxy-benzyl)-4-isobutyrylamino-pyrimidin-2-yl]-isobutyramide formula **3** (R = CH(CH₃)₂)
N-[4-(2,2-Dimethyl-propionylamino)-5-(2-iodo-3,4,5-trimethoxy-benzyl)-pyrimidin-2-yl]-2,2-dimethyl-propionamide formula **5** (R = C(CH₃)₃)
N-[5-(2-Iodo-3,4,5-trimethoxy-benzyl)-4-isobutyrylamino-pyrimidin-2-yl]-isobutyr-amide formula **5** (R = CH(CH₃)₂)
N-[4-(2,2-Dimethyl-propionylamino)-5-(2-formyl-3-hydroxy-4,5-dimethoxybenzyl)-pyrimidin-2-yl]-2,2-dimethyl-propionamide formula **6** (R = C(CH₃)₃)
N-[5-(2-Formyl-3-hydroxy-4,5-dimethoxy-benzyl)-4-isobubtyrylamino-pyrimidin-2-yl]-isobutyramide formula **6** (R = CH(CH₃)₂)
N-[4-(2,2-Dimethyl-proionylamino)-5-(2-formyl-4,5-dimethoxy-3-methoxymethoxy-benzyl)-pyrimidin-2-yl]-2,2-dimethyl-propionamide formula **7** (R = C(CH₃)₃)
N-[5-(2-Formyl-4,5-dimethoxy-3-methoxymethoxy-benzyl)-4-isobutyrylamino-pyrimidin-2-yl]-isobutyramide formula 7 (R = CH(CH₃)₂)
N-[5-(2-Formyl-4,5-dimethoxy-3-allyloxy-benzyl)-4-isobutyrylamino-pyrimidin-2-yl]-isobutyramide formula 7 (R = CH(CH₃)₂)
N-{5-[2-(3-Cyclopropyl-3-oxo-propenyl)-4,5-dimethoxy-3-methoxymethoxy-benzyl]-4-(2,2-dimethyl-propionylamino)-pyrimidin-2-yl}-2,2-dimethyl-propionamide formula 9 (R = C(CH₃)₃)
N-{5-[2-(3-Cyclopropyl-3-oxo-propenyl)-4,5-dimethoxy-3-methoxymethoxy-benzyl]-4-isobutyrylamino-pyrimidin-2-yl}-isobutyramide formula **9** (R = CH(CH₃)₂)
N-{5-[2-(3-Cyclopropyl-3-oxo-propenyl)-4,5-dimethoxy-3-allyloxy-benzyl]-4-isobutyrylamino-pyrimidin-2-yi}-isobutyramide formula **9** (R = CH(CH₃)₂)
N-{5-[2-(3-Cyclopropyl-3-hydroxy-propenyl)-4,5-dimethoxy-3-methoxymethoxy-benzyl]-4-(2,2-dimethyl-propionylamino)-pyrimidin-2-yl}-2,2-dimethyl-propionamide formula 10 (R = C(CH₃)₃)
N-{5-[2-(3-Cyclopropyl-3-hydroxy-propenyl)-4,5-dimethoxy-3-methoxymethoxy-benzyl]-4-isobutyrylamino-pyrimidin-2-yl}-isobutyramide formula **10** (R = CH(CH₃)₂)
N-{5-[2-(3-Cyclopropyl-3-hydroxy-propenyl)-4,5-dimethoxy-3-allyloxy-benzyl]-4-isobutyrylamino-pyrimidin-2-yl}-isobutyramide formula 10 (R = CH(CH₃)₂)
N-[5-(2-Cyclopropyl-7,8-dimethoxy-2H-chromen-5-ylmethyl)-4-(2,2-dimethyl-propionylamino)-pyrimidin-2-yl]-2,2-dimethyl-propionamide formula **11** (R = C(CH₃)₃)
N-[5-(2-Cyclapropyl-7,8-dimethoxy-2H-chromen-5-ylmethyl)-4-isabutyrylamino-pyrimidin-2-yl]-isobutyramide formulae **11** (R = CH(CH₃)₂)
N-[5-(2-Acetyl-3,4,5-trimethoxy-benzyl)-4-(2,2-dimethyl-propionylamino)-pyrimidin-2-yl]-2,2-dimethyl-propionamide formula **12** (R = C(CH₃)₃)
N-[5-(2-Acetyl-3,4,5-trimethoxy-benzyl)-4-isobutyrylamino-pyrimidin-2-yl]-isobutyr-amide formula **12** (R = CH(CH₃)₂)
N-[5-(2-Acetyl-3-hydroxy-4,5-dimethoxy-benzyl)-4-(2,2-dimethyl-propionylamino)-pyrimidin-2-yl]-2,2-dimethyl-propionamide formula **13** (R = C(CH₃)₃)
N-[5-(2-Acetyl-3-hydroxy-4,5-dimethoxy-benzyl)-4-isobutyrylamino-pyrimidin-2-yl]-isobutyramide formula **13** (R = CH(CH₃)₂)
N-[5-(2-Cyclopropyl-7,8-dimethoxy-4-oxo-chroman-5-ylmethyl)-4-(2,2-dimethyl-propionylamino)-pyrimidin-2-yl]-2,2-dimethyl-propionamide formula **15** (R = C(CH₃)₃)
N-[5-(2-Cyclopropyl-7,8-dimethoxy-4-oxo-chroman-5-ylmethyl)-4-isobutyrylamino-pyrimidin-2-yl]-isobutyramide formula **15** (R = CH(CH₃)₂)
N-{5-[2-(3-Cyclopropyl-acryloyl)-3,4,5-trimethoxy-benzyl]-4-(2,2-dimethyl-propionylamino)-pyrimidin-2-yl}-2,2-dimethyl-propionamide formula **17** (R = C(CH₃)₃)
N-{5-[2-(3-Cyclopropyl-acryloyl)-3,4,5-trimethoxy-benzyl]-4-isobutyrylamino-pyrimidin-2-yl}-isobutyramide formula **17** (R = CH(CH₃)₂)
N-{5-[2-(3-Cyclopropyl-3-iodo-propionyl)-3-hydroxy-4,5-dimethoxy-benzyl]-4-(2,2-dimethyl-propionylamino)-pyrimidin-2-yl}-2,2-dimethyl-propionamide formula **18** (R = C(CH₃)₃)
N-{5-[2-(3-Cyclopropyl-3-iodo-propionyl)-3-hydroxy-4,5-dimethoxy-benzyl]-4-isobutyrylamino-pyrimidin-2-yl}-isobutyramide formula **18** (R = CH(CH₃)₂)
N-[5-(2-Cyclopropyl-4-hydroxy-7.8-dimethoxy-chroman-5-ylmethyl)-4-(2,2-dimethyl-propionylamino)-pyrimidin-2-yl]-2,2-dimethyl-propionamide formula **19** (R = C(CH₃)₃)
N-[5-(2-Cyclopropyl-4-hydroxy-7,8-dimethoxy-chroman-5-ylmethyl)-4-isobutyrylamino-pyrimidin-2-yl]-isobutyramide formula **19** (R = CH(CH₃)₂)
